# NEW EUROPEAN PATENT SPECIFICATION

(11) **EP 0 430 491 B2**
(45) Date of publication and mention of the opposition decision: **25.10.2000**
(45) Mention of the grant of the patent: 08.06.1994
(21) Application number: 90312435.2
(22) Date of filing: 14.11.1990
(51) Int. Cl.: A61L 15/16

(54) **Transdermal delivery device for estradiol and process for manufacturing said device**
Gegenstand für die transdermale Applikation von Estradiol und Verfahren zur Herstellung eines solchen Gegenstandes
Dispositif pour l'administration transdermique d'estradiol et procédé pour la fabrication d'un tel dispositif

(30) Priority: 17.11.1989 AR 315479
(43) Date of publication of application: 05.06.1991
(73) Proprietor: Beta Pharmaceuticals Co., 1201 Montevideo (UY)
(72) Inventor: Stefano, Francisco José Evaristo, Buenos Aires (AR); Nowogrodski, José Adrián, Buenos Aires (AR); Carrara, Dario Norberto Ramon, Buenos Aires (AR)
(74) Representative: Paget, Hugh Charles Edward

(56) References cited:
- EP-A- 0 171 742
- EP-A- 0 261 429
- EP-A- 0 379 045
- WO-A-87/03490
- WO-A-90/06120
- BIOLOGICAL ABSTRACTS, vol 88, nr 1, 1989, ab- stract nr 9062, Philadelphia,PA, US; GOODMAN et al.: "Action of penetration enhancers on skin as assessed bythe permeation of model drug 5-fluorouracil and estradiol",& J. INVEST.DARMATOL. 91(4): 323-327. 1988
- YIE W. CHIEN: "Transdermal controlled systemic medications", Marcel Dekker, Inc. (1987), pp. 34, 36, 67-72, 78-81

## Description

The present invention relates to a device or system (hereinafter "device") for the administration of estradiol by the transdermal or percutaneous route, and to a process for manufacturing said device.

The transdermal or percutaneous administration of drugs provides a new and useful alternative to classical methods, and has been developed since 1960 (R.W. Baker and J. Farrant "Patents in transdermal drug delivery", Drug Delivery Systems 1987 Conference Proceedings) for the systematic treatment of various medical conditions to provide constant and efficient levels of nitroglycerin in the blood (USSN 806257 filed 6th November 1985, USSN 275215 filed 19th April 1985/85 and U.S. Patent No. 3996934); and also to.administer various types of steroids, such as ethinyl estradiol (EPA 0279982), estradiol (USSN 806257), and various anti-inflammatories (EPA 0267617), for example.

All known devices have in common a bandage or flexible substrate, a protective release film and a layer in which the active ingredient is dispersed in a vehicle which, in certain cases, contains adhesive components ("adhesive device") and in other cases an adhesive material is provided in an external layer and the active ingredient is contained in a separate layer or intermediate matrix (these devices are called respectively "monolithic devices" and "reservoir devices", see R.W. Baker et. al, supra).

With the devices proposed and developed in the prior art, adjuvants known as "increasers", "enhancers" or "permeation enhancers" are also included for increasing the rate of permeation of the active ingredient. In addition to dimethylsulfoxide and azone (1-dodecylhexahydro-2H-azepin-2-one) (EP-A-0251425), ethanol, propylene glycols, other alcohols, long-chained fatty acids (and esters thereof) etc., have been also proposed ( R.W. Baker et al, supra ).

It has now been found that a further transdermal delivery device for estradiol can be obtained and is suitable for the treatment of side effects and menopausal disorders such as hot flushes, depression, osteoporosis, having rapid and sustained effects depending on the use of a binary "enhancer" component including a combination of at least one compound selected from unsaturated fatty acids (C₁₂-C₁₈) and alkyl esters thereof with at least one compound selected from glycerin and (C₂-C₄)alkylene-1,2-glycols.

It is an object of the present invention to provide a transdermal delivery device for the transdermal administration of estradiol (SAT) which allows a high transdermal flux with which it is possible to maintain estradiol at the required constant high level in the blood.

This invention further relates to a transdermal delivery device for estradiol whereby metabolic degradation of estradiol to estrone during permeation is inhibited to a large extent.

In one aspect, the present invention provides a transdermal delivery device for estradiol as set out in claim 1.

The estradiol is preferably present in an amount exceeding its saturation concentration in said coating layer.

In another aspect, the present invention provides a process for manufacturing the transdermal delivery device for estradiol, as set out in claim 9. Further the invention in another aspect consists in the use of certain compounds as set out in claim 12.

Preferably, the amount of estradiol in said coating layer is in excess of the saturation concentration therein.

Conveniently, the coating layer is provided by coating it on the releasable protective layer, eg a silicone layer, bringing the flexible substrate into contact with the coating layer, and then cutting the resultant structure to the required shape and size.

The releasable protective layer is discarded immediately before the device is applied to the skin of a patient. Obviously, the formulation of the coating layer, particularly the adhesive material, is not only dermatologically acceptable but also pharmacologically acceptable.
i) The adhesive material may be selected from a wide variety of pressure-sensitive adhesive materials such as silicones, rubber, PIB (polyisobutylene) and acrylic adhesives.
ii) A reinforcing component (solid) which may be selected from the polyterpene resins, such as Piccolyte S115 (a terpene resin), Wingtack 115, modified colophony resins such as Pentalyn (an esterified colophony resin with pentaerythritol), etc. may also be included.

Amongst materials suitable for the flexible substrate, cellophane (cellulose xanthate film), Saran (polyvinylidene chloride film), polyvinyl chloride coatings, polyesters such as polyethylene terephthalate including binary structures such as aluminium-polyethylene coatings, polyester-polyethylene coatings, etc, may be used.

For the releasable protective layer, any of the above-mentioned coatings for the substrate can be used, preferably polyesters, such as polyethylene terephthalate, etc covered with a silicone to prevent sticking of the adhesive.

An amount of estradiol in the adhesive matrix of from about 1 to 5 per cent, preferably 2 per cent, by weight based on the total weight of the coating layer adhesive matrix, is sufficient for the purposes of this invention. The amount of estradiol preferably exceeds the saturation concentration thereof in said coating layer in order to keep the flux at a constant rate and thereby maintain a more constant level of estradiol in the blood. Indeed, it is possible to formulate coating layers without reaching saturation levels if the required dosage and the estradiol level so requires it.

The estradiol permeation enhancing component performs a vital role in the maintenance of constant systemic levels of estradiol.

The above mentioned alkylene diols and, particularly propylene glycol, have been proved to be an inhibitor of the skin metabolism degradation of estradiol to estrone, that is to say, the skin not only has a passive role recognized as protector against the penetration of exogenous agents, since it has been demonstrated as being a metabolizer of certain substances, among them estradiol which undergoes enzymatic degradation to estrone.

Tests conducted by the applicant have shown that propyleneglycol inhibits the enzymatic mechanism that causes the degradation of estradiol to estrone. Therefore, the propyleneglycol added (and other low chain alkylenediols) complements the enhancer effect of the fatty acids by avoiding or reducing estradiol degradation, thereby enabling systemic estradiol levels to be obtained which are higher than those obtainable by conventional transdermal delivery devices which operate without blocking said enzymatic mechanism, but which use, for example, oleic acid as "enhancer", as will be appreciated from Table 4 hereinafter.

The present invention will be further described in the following Examples

### EXAMPLE 1

### Preparation of formulas containing estradiol.

### A) Preparation of an adhesive mixture.

To a vinyl acetate/acrylate polymer solution diluted with ethanol/toluene/ethyl acetate to 30% solids, a solid rosin tackifier component (Pentalyn A) is added with stirring at room temperature for a period of time necessary to obtain a homogeneous mixture (for about 4 hours)

### B) Preparation of formula containing estradiol.

To the material prepared in step A) above, whilst stirring, estradiol and the permeation rate enhancer (a mixture of oleic acid and propylene glycol) are added. A clear solution is obtained and kept in a closed vessel to avoid evaporation of the solvent medium until air bubbles have dispersed. Finally, the solution is filtered through a stainless sieve (100 mesh).

### C) Preparation of the transdermal delivery device.

On a flexible laminate (which later defines the releasable protective layer), the solution prepared as described above, is applied by means of a conventional coating device. Said solution is applied to one of the surfaces of the flexible laminate (which may, for example, be a polyester film previously sprayed with silicone) to form a layer of predetermined thickness, for example, 400 micrometres, and is subsequent dried by hot air circulation.

The thus-coated siliconised polyester is laminated on a second flexible film which constitutes the substrate in the finished device.

The process ends with cutting to size, for example, by means die cutting of the multi-layer laminate to form shapes of the desired geometry and size.

### TRANSDERMAL PERMEABILITY AND ESTRADIOL BIOAVAILABILITY OF TRANSDERMAL ADMINISTRATION ESTRADIOL

The rate of estradiol permeation in the formulations according to the present invention, was examined "in vivo" by measuring the estradiol blood level in 6 voluntary post-menopausal women, using devices of different surfaces as described in Example 1, estradiol concentration being measured in the blood plasma 24 hours after application.

The Table 1 below illustrates the result obtained

**Table 1**

| Device surface (cm²) | Estradiol level in plasma (pg/ml) |
|---|---|
| 8.5 | 20.5 ± 2.4 |
| 17 | 50.5 ± 9.1 |
| 35 | 121.8 ± 37.2 (means values ± SEM) |

The experiment was repeated with the device of Example 1 cut to 17cm² to verify the amount of estradiol accumulated in the blood plasma, in assays lasting 72 hours.

**TABLE 2**

| Time | Estradiol level (pg/ml) |
|---|---|
| 24 hours | 50.50 ± 9.11 |
| 48 hours | 62.75 ± 11.76 |
| 72 hours | 48.34 ± 6.55 (mean values ± SEM) |

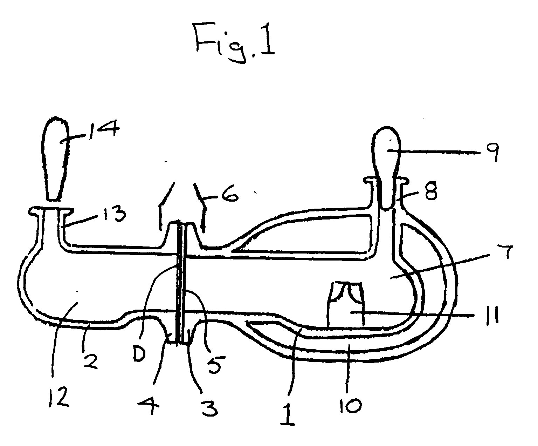

Furthermore, "in vitro" permeation experiments with abdominal mouse skin and human skin were made using the diffusion chamber that is schematically shown in longitudinal section in Fig. 1.

The diffusion chamber illustrated in Fig. 1 comprises a body having front and rear tubular sections 1 and 2 having respective annular end flanges 3 and 4 by which the sections 1 and 2 are joined together. A sample of skin 5, with attached estradiol delivery device D to be tested facing the rear tubular section 2, is clamped between the flanges 3 and 4 by means of a pinch clamp 6.

The tubular section 1 has an internal chamber 7 with an opening 8 furnished with a glass stopper 9. The tubular section 1 also has a jacket 10 through which warm water can be circulated to maintain the contents of the chamber 7 at the desired temperature. The chamber 7 contains a magnetic bar stirrer 11 which is rotatable by means of a magnetic field applied by external means (not shown) to enable the contents of the chainber 7 to be stirred.

The tubular section 2 has a chamber 12 with an opening 13 furnished with a glass stopper 14.

In the assay, the skin of Swiss mice, shaved 72 hours before the experiments, was employed. Also used-was human stratum corneum obtained from human skin samples (remnants of plastic surgery) which had been treated by immersion in water at 60°C for a minute in order to separate the strateum corneum therefrom by exfoliation.

Both the hairless mouse skin and the human stratum corneum with estradiol delivery device attached were clamped between the flanges 3 and 4, as shown in Fig. 1, with the dermis towards chamber 12 which is a receptor chamber. Each experiment was started by loading chamber 7 with 5.5 ml of a receptor solution of sodium laurylsulfate (0.5 %), starting the stirrer 11 and taking samples at pre-determined periods of time and the subsequent determination of permeated estradiol (by HPLC).

The following illustrates the results obtained in assays performed under the above mentioned conditions using devices as described in Example 1.

**Table 3**

| | Flux (µg/cm²/hour) | | | Accumulated Estradiol Permeation (µg/cm²) | | |
|---|---|---|---|---|---|---|
| | 5 h | 24 h | 48 h | 5 h | 24 h | 48 h |
| Mouse skin | 1.28 | 1.07 | 2.56 | 6.39 | 26.59 | 57.88 |
| Human Streteum Corneum | 0.24 | 0.64 | 0.88 | 1.18 | 13.94 | 48.91 |

The inhibition of the mechanism of the enzymatic degradation from estradiol to estrone to propyleneglycol has been studied using the same device as illustrated in Fig.1 as follows:

### INHIBITION OF THE ESTRADIOL METABOLISM IN HUMAN SKIN

Strateum corneum was eliminated by scraping with a surgical knife at 37°C
CONTROL: Saturated solution of estradiol in saline solution.
PG: Saturated solution of estradiol in saline solution + 10% PG.
The results of two sets of experiments are shown (n=3 each).

**TABLE 4**

| | Estradiol (nmol/ml) | Estrone (nmol/ml) | %Estradiol metabolized (E₁.100//E₂+E₁) | %Inhib |
|---|---|---|---|---|
| Exp. 1 | | | | |
| Control | 2.62 | 0.73 | 21.8 | 66.0 |
| PG | 3.51 | 0.27 | 7.4 | |

| Exp. 2 | | | | |
|---|---|---|---|---|
| Control | 2.94 | 0.53 | 15.3 | 57.2 |
| PG | 2.71 | 0.19 | 6.55 | |

This shows estradiol metabolism in human skin. Under the control conditions the metabolite, estrone, was between 21.8 and 15.3% of the estradiol present in the incubation bath, while with PG 10% the conversion was inhibited by 57- 63%. In mouse skin, the controls resulted in a change of 7-15% suffering an inhibition to 1-2% with PG.

## Claims

1. A transdermal delivery device for estradiol comprising a flexible substrate and a releasable protective film, the protected surface of said flexible substrate being at least partly covered with a coating layer containing (a) a dermatologically acceptable pressure sensitve adhesive material for securing the device to the skin, (b) estradiol homogeneously distributed as a pharmacologically active ingredient and (c) a component which enhances the rate of estradiol permeation and which comprises from 1 to 20 wt% of at least one first compound selected from unsaturated (C₁₂-C₁₈) fatty acids and alkyl esters thereof, and from 5 to 30 wt% of at least one second compound selected from glycerin and (C₃-C₆)alkylene 1,2-diols, the percentages being based on the total weight of said coating layer, wherein the releasable protective film, which lies on the opposite side of the coating layer to the flexible substrate, is formed of any one of
cellulose xanthate film,
polyvinylidene chloride film.
polyvinyl chloride,
polyester,
a binary structure of aluminium-polyethylene, and
a binary structure of polyester - polyethylene,
and is covered with a silicone.

2. A device according to claim 1, wherein the amount of estradiol in the coating layer exceeds the saturation concentration thereof.

3. A device according to claim 1 or 2, wherein said coating layer comprises in an homogeneous mixture:
i) a polymer vehicle selected from silicones, acrylic or methacrylic polymers, natural or synthetic rubber and mixtures thereof;
ii) an adhesive material selected from polyterpenes, modified colophony resins and mixtures thereof.

4. A device according to claim 1, 2 or 3, wherein said component comprises oleic acid and propyleneglycol in a ratio of 1:0.5 to 1:5.

5. A device according to claim 4, wherein the ratio is 1:1.5.

6. A device according to any preceding claim, wherein said flexible substrate is of cellulose xanthate, aluminium, a polymer or a laminate of any two or more thereof.

7. A device according to claim 6, wherein the polymer is a polyvinyl chloride, polyvinylidene or polyethylene.

8. A device according to any preceding claim, wherein the amount of estradiol in the coating layer is sufficient to maintain its concentration at least at saturation level during predetermined periods of time.

9. A process for manufacturing a transdermal delivery device for estradiol, comprising the steps of providing a coating layer on at least part of one surface of a flexible substrate, said coating layer containing (a) a dermatologically acceptable pressure sensitive adhesive material for securing the device to the epidermis, (b) estradiol homogeneously distributed as a pharmacologically active ingredient and (c) a component which enhances the rate of estradiol permeation and which comprises from 1 to 20 wt% of at least one first compound selected from unsaturated (C₁₂-C₁₈) fatty acids and alkyl esters thereof, and from 5 to 30 wt% of at least one second compound selected from glycerin and (C₃-C₆)alkylene 1,2-diols, the percentages being based on the total weight of said coating layer, and providing a releasable protective layer which, in the finished device, lies on the opposite side of the coating layer to the flexible substrate, wherein said releasable protective film is formed of any one of
cellulose xanthate film,
polyvinylidene chloride film.
polyvinyl chloride.
polyester,
a binary structure of aluminium-polyethylene, and
a binary structure of polyester - polyethylene,
and is covered with a silicone.

10. A process according to claim 9, wherein the amount of estradiol in said coating layer is in excess of the saturation concentration therein.

11. A process according to claim 9 or 10, wherein the coating layer is provided by coating it on the releasable protective layer, bringing the flexible substrate into contact with the coating layer, and then cutting the resultant structure to the required shape and size.

12. The use of at least one compound selected from glycerin and (C₃-C₆) alkylene-1,2-diols for inhibiting metabolic degradation of estradiol to estrone during permeation in a transdermal delivery system for estradiol comprising a flexible substrate and a releasable protective film, the protected surface of said flexible substrate being at least partly covered with a coating layer containing (a) a dermatologically acceptable pressure sensitive adhesive material for securing the device to the skin, (b) estradiol homogeneously distributed as a pharmacologically active ingredient and (c) a component which enhances the rate of estradiol permeation and which comprises 1 to 20 wt% of at least one compound selected from unsaturated (C₁₂-C₁₈) fatty acids and alkyl esters thereof and from 5 to 30 wt% of said at least one compound selected from glycerin and (C₃-C₆) alkylene 1,2-diols, said percentages being based on the total weight of said coating layer.

13. The use according to claim 12, wherein the amount of estradiol in the coating layer exceeds the saturation concentration thereof.

14. The use according to claim 12 or 13, wherein said coating layer comprises in an homogeneous mixture:
(ii) a polymer vehicle selected from silicones, acrylic or methacrylic polymers, natural or synthetic rubber and mixtures thereof;
(ii) an adhesive material selected from polyterpenes, modified colophony resins and mixtures thereof.

15. The use according to claim 12, 13 or 14, wherein said component comprises oleic acid and propyleneglycol in a ratio of 1:0.5 to 1:5.

16. The use according to claim 15, wherein the ratio is 1:1.5.

17. The use according to any one of claims 12 to 16, wherein said flexible substrate is of cellulose xanthate, aluminium, a polymer or a laminate of any two or more thereof.

18. The use according to claim 17, wherein the polymer is a polyvinyl chloride, polvinylidene or polyethylene.

19. The use according to any one of claims 12 to 18, wherein the amount of estradiol in the coating layer is sufficient to maintain its concentration at least at saturation level during predetermined periods of time.

## Patentansprüche

1. Vorrichtung zur transdermalen Applikation von Östradiol, die umfaßt ein flexibles Substrat und einen abziehbaren Schutzfilm, wobei die geschützte Oberfläche des genannten flexiblen Substrats mindestens teilweise von einer Überzugsschicht bedeckt ist, die enthält (a) ein dermatologisch akzeptables druckempfindliches (selbstklebendes) Klebstoffmaterial zum Befestigen der Vorrichtung an der Haut, (b) Östradiol, das als pharmakologisch aktiver Bestandteil (Wirkstoff) homogen darin verteilt ist, und (c) eine Komponente zur Verbesserung der Östradiol-Permeations-Rate, die umfaßt 1 bis 20 Gew.-% mindestens einer ersten Verbindung, ausgewählt aus ungesättigten (C₁₂-C₁₈)-Fettsäuren und ihren Alkylestern, und 5 bis 30 Gew.-% mindestens einer zweiten Verbindung, ausgewählt aus Glycerin und (C₃-C₆)-Alkylen-1,2-diolen, wobei die Prozentangaben jeweils bezogen sind auf das Gesamtgewicht der genannten Überzugsschicht,
wobei der abziehbare Schutzfilm auf der der Überzugsschicht gegenüberliegenden Seite des flexiblen Substrats angeordnet ist und gebildet ist aus einem Vertreter aus der Gruppe
Cellulosexanthat-Film,
Polyvinylidenchlorid-Film,
Polyvinylchlorid,
Polyester,
einer binären Aluminium-Polyethylen-Struktur und
einer binären Polyester-Polyethylen-Struktur,
und mit einem Silicon bedeckt ist.

2. Vorrichtung nach Anspruch 1, worin die Menge des Östradiols in der Überzugsschicht dessen Sättigungskonzentration übersteigt.

3. Vorrichtung nach Anspruch 1 oder 2, worin die Überzugsschicht in Form einer homogenen Mischung umfaßt:
i) ein Polymer-Vehiculum, ausgewählt aus Siliconen, Acryl- oder Methacryl-Polymeren, natürlichem oder synthetischem Kautschuk und Mischungen davon; und
ii) ein Klebstoffmaterial, ausgewählt aus Polyterpenen, modifizierten Kolophoniumharzen und Mischungen davon.

4. Vorrichtung nach Anspruch 1, 2 oder 3, worin die genannte Komponente Ölsäure und Propylenglycol in einem Verhältnis von 1:0,5 bis 1:5 umfaßt.

5. Vorrichtung nach Anspruch 4, worin das Verhältnis 1:1,5 beträgt.

6. Vorrichtung nach irgendeinem vorhergehenden Anspruch, worin das genannte flexible Substrat aus Cellulosexanthat, Aluminium, einem Polymer oder einem Laminat von jeweils 2 oder mehr derselben besteht.

7. Vorrichtung nach Anspruch 6, worin das Polymer ein Polyvinylchlorid, Polyvinyliden oder Polyethylen ist.

8. Vorrichtung nach irgendeinem vorhergehenden Anspruch, worin die Menge des Östradiols in der Überzugsschicht ausreicht, um seine Konzentration während vorgegebener Zeitspannen mindestens bei dem Sättigungswert zu halten.

9. Verfahren zur Herstellung einer Vorrichtung zur transdermalen Applikation von Östradiol, das die folgenden Stufen umfaßt:
Aufbringen einer Überzugsschicht auf mindestens einen Teil einer Oberfläche eines flexiblen Substrats, wobei diese Überzugsschicht enthält (a) ein dermatologisch akzeptables druckempfindliches (selbstklebendes) Klebstoffmaterial zum Befestigen der Vorrichtung an der Epidermis, (b) Östradiol, das als pharmakologisch aktiver Bestandteil (Wirkstoff) homogen darin verteilt ist, und (c) eine Komponente, welche die Östradiol-Permeations-Rate verbessert und umfaßt 1 bis 20 Gew.-% mindestens einer ersten Verbindung, ausgewählt aus ungesättigten (C₁₂-C₁₈)-Fettsäuren und ihren Alkylestern, und 5 bis 30 Gew.-% mindestens einer zweiten Verbindung, ausgewählt aus Glycerin und (C₃-C₆)-Alkylen-1,2-diolen, wobei die Prozentangaben auf das Gesamtgewicht der Überzugsschicht bezogen sind, und
Aufbringen einer abziehbaren Schutzschicht, die in der fertigen Vorrichtung auf der der Überzugsschicht gegenüberliegenden Seite des flexiblen Substrats liegt,
wobei der genannte abziehbare Schutzfilm gebildet ist aus einem Vertreter aus der Gruppe
Cellulosexanthat-Film,
Polyvinylidenchlorid-Film,
Polyvinylchlorid,
Polyester,
einer binären Aluminium-Polyethylen-Struktur und
einer binären Polyester-Polyethylen-Struktur,
und mit einem Silicon bedeckt ist.

10. Verfahren nach Anspruch 9, worin die Menge des Östradiols in der genannten Überzugsschicht dessen Sättigungskonzentration darin übersteigt.

11. Verfahren nach Anspruch 9 oder 10, worin die Überzugsschicht aufgebracht wird durch Beschichten der abziehbaren Schutzschicht damit durch Inkontaktbringen des flexiblen Substrats mit der Überzugsschicht und anschließendes Zuschneiden der resultierenden Struktur auf die erforderliche Gestalt und Größe.

12. Verwendung mindestens einer Verbindung, ausgewählt aus Glycerin und (C₃-C₆)-Alkylen-1,2-diolen, zur Inhibierung des Stoffwechsel-Abbaus von Östradiol zu Östron während der Permeation in einem System zur transdermalen Applikation von Östradiol, das umfaßt ein flexibles Substrat und einen abziehbaren Schutzfilm, wobei die geschützte Oberfläche des genannten flexiblen Substrats mindestens teilweise von einer Überzugsschicht bedeckt ist, die enthält (a) ein dermatologisch akzeptables druckempfindliches (selbstklebendes) Klebstoffmaterial zum Befestigen der Vorrichtung an der Haut, (b) Östradiol, das als pharmakologisch aktiver Bestandteil (Wirkstoff) homogen darin verteilt ist, und (c) eine Komponente zur Verbesserung der Östradiol-Permeations-Rate und die umfaßt 1 bis 20 Gew.-% mindestens einer Verbindung, ausgewählt aus ungesättigten (C₁₂-C₁₈)-Fettsäuren und ihren Alkylestern, und 5 bis 30 Gew.-% mindestens einer Verbindung, ausgewählt aus Glycerin und (C₃-C₆)-Alkylen-1,2-diolen, wobei die Prozentangaben jeweils bezogen sind auf das Gesamtgewicht der Überzugsschicht.

13. Verwendung nach Anspruch 12, worin die Menge des Östradiols in der Überzugsschicht dessen Sättigungskonzentration übersteigt.

14. Verwendung nach Anspruch 12 oder 13, worin die genannte Überzugsschicht in Form einer homogenen Mischung umfaßt:
i) ein Polymer-Vehiculum, ausgewählt aus Siliconen, Acryl- oder Methacryl-Polymeren, natürlichem oder synthetischem Kautschuk und Mischungen davon; und
ii) ein Klebstoffmaterial, ausgewählt aus Polyterpenen, modifizierten Kolophoniumharzen und Mischungen davon.

15. Verwendung nach Anspruch 12, 13 oder 14, worin die genannte Komponente Ölsäure und Propylenglycol in einem Verhältnis von 1:0,5 bis 1:5 umfaßt.

16. Verwendung nach Anspruch 15, worin das Verhältnis 1:1,5 beträgt.

17. Verwendung nach einem der Ansprüche 12 bis 16, worin das genannte flexible Substrat aus Cellulosexanthat, Aluminium, einem Polymer oder einem Laminat von jeweils 2 oder mehr derselben besteht.

18. Verwendung nach Anspruch 17, worin das Polymer ein Polyvinylchlorid, Polyvinyliden oder Polyethylen ist.

19. Verwendung nach einem der Ansprüche 12 bis 18, worin die Menge des Östradiols in der Überzugsschicht ausreicht, um seine Konzentration während vorgegebener Zeitspannen mindestens bei dem Sättigungswert zu halten.

## Revendications

1. Dispositif de délivrance transdermique pour l'oestradiol comprenant un substrat flexible et un film protecteur libérable, la surface protégée dudit substrat flexible étant au moins en partie recouverte par une couche d'enrobage contenant (a) un matériau adhésif sensible à la pression dermatologiquement acceptable pour retenir le dispositif à la peau, (b) de l'oestradiol distribué de façon homogène en tant qu'ingrédient pharmacologiquement actif et (c) un composé qui accroît le taux de perméation de l'oestradiol et qui comprend de 1 à 20 % en poids d'au moins un premier composé choisi parmi les acides gras (C₁₂-C₁₈) insaturés et leurs alkyl esters, et de 5 à 30 % en poids d'au moins un second composé choisi parmi la glycérine et des alkylènes 1,2-diols (C₃- C₆), les pourcentages étant exprimés par rapport au poids total de ladite couche d'enrobage, dispositif dans lequel le film protecteur libérable qui repose sur la face opposée de la couche d'enrobage au substrat flexible est formé par
un film de xanthate de cellulose,
un film de chlorure de polyvinylidène,
un chlorure de polyvinyle,
du polyester,
une structure binaire d' aluminium-polyéthylène et
une structure binaire de polyester-polyéthylène,
et est recouvert d'une silicone.

2. Dispositif selon la revendication 1, dans lequel la teneur en oestradiol dans la couche d'enrobage excède sa concentration de saturation.

3. Dispositif selon la revendication 1 ou 2, dans lequel ladite couche d'enrobage comprend un mélange homogène :
i) d'un polymère véhicule choisi parmi des silicones, des polymères acryliques ou méthacryliques, un caoutchouc naturel ou synthétique et leurs mélanges ;
ii) d'un matériau adhésif choisi parmi les polyterpènes, les résines de colophane modifiées et leurs mélanges.

4. Dispositif selon la revendication 1, 2 ou 3, dans lequel ledit composé comprend de l'acide oléique et du propylèneglycol dans un rapport de 1:0,5 à 1:5.

5. Dispositif selon la revendication 4, dans lequel le rapport est 1:1,5.

6. Dispositif selon l'une quelconque des revendications précédentes dans lequel ledit substrat flexible est du xanthate de cellulose, de l'aluminium, un polymère ou un stratifié de deux ou plus de ceux-ci.

7. Dispositif selon la revendication 6, dans lequel le polymère est un chlorure de polyvinyle, un polyvinilydène ou un polyéthylène.

8. Dispositif selon l'une quelconque des revendications précédentes, dans lequel la teneur en oestradiol dans la couche d'enrobage est suffisante pour maintenir sa concentration à au moins le niveau de saturation durant des périodes de temps prédéterminées.

9. Procédé pour la préparation d'un dispositif de délivrance transdermique pour l'oestradiol, comprenant les étapes consistant à disposer une couche d'enrobage à au moins une partie d'une surface du substrat flexible, ladite couche d'enrobage contenant (a) un matériau adhésif sensible à la pression dermatologiquement acceptable pour retenir le dispositif sur l'épiderme, (b) de l'oestradiol distribué de façon homogène en tant qu'ingrédient actif pharmacologiquement et (c) un composé qui accroît le taux de perméation de l'oestradiol et qui comprend de 1 à 20 % en poids d'au moins un premier composé choisi parmi les acides gras (C12-C18) insaturés et leurs alkyl esters, et de 5 à 30 % en poids d'au moins un second composé choisi parmi la glycérine et des alkylènes 1,2-diols (C3-C6), les pourcentages étant exprimés par rapport au poids total de ladite couche d'enrobage; et disposer une couche protectrice libérable qui, dans le dispositif fini, repose sur la phase opposée de la couche d'enrobage au substrat flexible, procédé dans lequel le film protecteur libérable est formé par
un film de xanthate de cellulose,
un filin de chlorure de polyvinylidène,
un chlorure de polyvinyle,
du polyester,
une structure binaire d'aluminium-polyéthylène et
une structure binaire de polyester-polyéthylène,
et est recouvert d'une silicone.

10. Procédé selon la revendication 9, dans lequel la teneur en oestradiol dans ladite couche d'enrobage est en excès par rapport à la concentration de saturation.

11. Procédé selon la revendication 9 ou 10, dans lequel la couche d'enrobage est obtenue en l'enrobant sur la couche protectrice libérable, en mettant en contact le substrat flexible avec la couche d'enrobage, et ensuite en coupant la structure résultante à la forme et à la taille désirée.

12. Utilisation d'au moins un composé choisi parmi la glycérine et les alkylènes 1,2-diols (C3-C6)pour inhiber la dégradation métabolique de l'oestradiol en oestrone durant la perméation dans un dispositif de délivrance transdermique pour l'oestradiol comprenant un substrat flexible et un film protecteur libérable, la surface protégée du dit substrat flexible étant au moins partiellement recouverte d'une couche de revêtement contenant (a) un matériau adhésif sensible à la pression dermatologiquement acceptable pour retenir le dispositif sur la peau (b) de l'oestradiol distribué de façon homogène en tant qu'ingrédient pharmacologiquement actif et (c) un composé qui accroît le taux de perméation de l'oestradiol et qui comprend de 1 à 20 % en poids d'au moins un premier composé choisi parmi les acides gras (C₁₂-C₁₈) insaturés et leurs alkyl esters, et de 5 à 30 % en poids d'au moins un second composé choisi parmi la glycérine et des alkylènes 1,2-diols (C₃- C₆), les pourcentages étant exprimés par rapport au poids total de ladite couche d'enrobage,

13. Utilisation selon la revendication 12 dans laquelle la teneur en oestradiol dans la couche d'enrobage excède sa concentration de saturation.

14. Utilisation selon la revendication 12 ou 13 dans laquelle ladite couche d'enrobage comprend un mélange homogène :
i) d'un polymère véhicule choisi parmi des silicones, des polymères acryliques ou méthacryliques, un caoutchouc naturel ou synthétique et leurs mélanges ;
ii) d'un matériau adhésif choisi parmi les polyterpènes, les résines de colophane modifiées et leurs mélanges.

15. Utilisation selon la revendication 12, 13 ou 14 dans laquelle ledit composé comprend de l'acide oléique et du propylèneglycol dans un rapport de 1:0,5 à 1:5.

16. Utilisation selon la revendication 15 dans laquelle le rapport est 1: 1,5.

17. Utilisation selon l'une quelconque des revendications 12 à 16 dans laquelle ledit substrat flexible est du xanthate de cellulose, de l'aluminium, un polymère ou un stratifié de deux ou plus de ceux-ci.

18. Utilisation selon la revendication 17 dans laquelle le polymère est un chlorure de polyvinyle, un polyvinylidène ou un polyéthylène.

19. Utilisation selon l'une quelconque des revendications 12 à 18 dans laquelle la teneur en oestradiol dans la couche d'enrobage est suffisante pour maintenir sa concentration à au moins le niveau de saturation durant des périodes de temps prédéterminées.
